Europäisches Patentamt

(19) European Patent Office

Office européen des brevets

(11) Publication number: **0 292 326**
**A2**

(12) **EUROPEAN PATENT APPLICATION**

(21) Application number: 88304626.0

(22) Date of filing: 20.05.88

(51) Int. Cl.⁴: **C 12 N 15/00**
C 12 N 9/72, C 12 N 9/48,
A 61 K 37/54

(30) Priority: **20.05.87 GB 8711957**

(43) Date of publication of application:
**23.11.88 Bulletin 88/47**

(84) Designated Contracting States:
**BE CH DE FR GB IT LI NL**

(71) Applicant: **BEECHAM GROUP PLC**
**Beecham House Great West Road**
**Brentford Middlesex TW8 9BD (GB)**

(72) Inventor: **Browne, Michael Joseph Beecham**
**Pharmaceuticals**
**Great Burgh Yew Tree Bottom Road**
**Epsom Surrey KT18 5XQ (GB)**

**Lawrence, Geoffrey Mark Prouse Beecham Pharm.**
**Great Burgh Yew Tree Bottom Road**
**Epsom Surrey KT18 5XQ (GB)**

(74) Representative: **Valentine, Jill Barbara et al**
**Beecham Pharmaceuticals Patents & Trade Marks Dept.**
**Great Burgh Yew Tree Bottom Road**
**Epsom Surrey KT18 5XQ (GB)**

The title of the invention has been amended (Guidelines for Examination in the EPO, A-III, 7.3).

(54) **Modified enzyme.**

(57) A hybrid plasminogen activator which comprises one to five kringle domains derived from plasminogen linked to the N-terminus of a t-PA species comprising at least a finger domain and a B-chain derived from t-PA.

## Description

## Novel Compounds

The present invention relates to a hybrid fibrinolytic enzyme, its preparation, pharmaceutical compositions containing it and its use in the treatment of thrombotic disease.

The sequence of amino acids making up the enzyme tissue-type plasminogen activator (t-PA) and the nucleotide sequence for the cDNA which codes for t-PA are known (see Pennica et al., 1983; Nature, 301, 214). t-PA is known to have fibrinolytic activity.

As used herein, the term tissue-type plasminogen activator (t-PA) denotes a plasminogen activator of the group having the immunological properties defined for t-PA at the XXVIII Meeting of the International Committee on Thrombosis and Haemostasis, Bergamo, Italy, 27 July 1982.

The amino acid sequence of various native forms of t-PA are known. The abovementioned Nature 1983 reference discloses the sequence for the L-chain and the mature S-chain forms of t-PA, also discussed by Vehar et.al., Biotechnology, 1984, 2, 1051-7 in which the processing of initially formed t-PA by removal of a pro-sequence to give the S-chain form is reported. Pohl et.al., FEBS letters, 1984, Vol. 168 No.1, 29-32, refers to the N-terminal multiplicity of t-PA and discloses the U-chain form. The numbering system for the amino acid sequence of t-PA used herein is that described in the Nature 1983 reference for mature (S-chain) t-PA in which the N-terminal serine is numbered 1. By this system, L-chain t-PA has an N-terminal glycine residue at position -3 and U-chain t-PA has an N-terminal valine at position 4. References to t-PA herein are understood to include all such native variant forms.

Native t-PA is composed of a B or light (L) and an A or heavy (H) chain. The B chain contains the active site of the enzyme.

It has been shown (Ny, T. et al, 1984; Proc. Natl. Acad. Sci. U.S.A., 81, 5355) that the A chain exhibits a number of structural and functional domains which are homologous to structures found in other plasma proteins: two triple disulphide-bonded structures or kringles, a growth-factor-like domain and a fibronectin-finger-like domain.

The region from amino acid residues 44 to 91 has been termed the "growth factor domain" (Banyai, L., et al FEBS Lett. 163, 37, 1983). The genetic information which codes for the major part of this domain, residues 51 to 86 inclusive, and partially codes for residues 50 and 87, lies on a single exon. The region from the first to last cysteine residues within this region, residues 51 to 84 inclusive, defines a triple-disulphide linked structure which will be referred to herein as the growth factor domain.

The region from the first to the last cysteine residue of the first kringle structure, residues 92 to 173 inclusive, will be referred to herein as the kringle 1 ($K_1^t$) domain.

The region from the first to the last cysteine residue of the second kringle structure, residues 180 to 261 inclusive, will be referred to herein as the kringle 2 ($K_2^t$) domain.

The region from the first to the last cysteine residue of the fibronectin-finger-like domain, residues 6 to 43, will be referred to herein as the finger (F) domain.

In addition to the native forms of t-PA described above, various t-PA muteins are also known, see for example EP-A-0201153, EP-A-0207589, EP-A-0233013, EP-A-0240334, EP-A-0241208, EP-A-0241209, EP-A-0241210, WO-86/01538, EP-A-0227462, WO-87/03906, WO-87/04722, EP-A-0242836, AU-8661804, EP-A-0234051, EP-A-0199574, EP-A-0253582, EP-A-0238304, EP-A-0225286, EP-A-0236289 and DE 3537176.

References herein to t-PA species include both native forms and muteins.

Plasmin is a two-chain serine protease which may be obtained by the cleavage of the single chain precursor, plasminogen, at a specific internal peptide bond. The amino acid sequence of human plasminogen is known (Wiman and Walters (1975) Eur.J. Biochem. 50, 489-494 and 58, 539-547; Wiman (1977) Eur. J. Biochem. 76, 129-137; Sottrup-Jensen et al. (1978) Fibrinolysis and Thrombolysis Vol. 3, 191-209, Raven Press, New York; and Sottrup-Jensen et al. (1978) Atlas of Protein Sequence and Structure Vol. 5, Suppl. 3, p91, National Biomedical Research Foundation, Silver Spring, MD). A partial nucleotide sequence coding for amino acid residues 272-790 of human plasminogen has also been described (Malinowski, D.P. et al., 1984, Biochemistry, 23, 4243-4250). The cleavage site of human plasminogen is located between residues arg-560 and val-561 (according to the sequence numbering of Sottrup-Jensen et al. (1978) Atlas of Protein Sequence (op.cit.)). Two species of plasminogen have been identified ( F.J. Castellino, Chemical Reviews Vol. 81 p431 (1981)): $Glu_1$ which has an N-terminal glutamic acid residue at position 1 and $lys_{77}$ which has an N-terminal lysine residue at position 77. Glu-plasminogen is also easily converted by limited plasmic digestion to other modified forms with N-terminal valine ($val_{78}$) or methionine ($met_{68}$) (C. Miyashita, E. Wenzel and M. Heiden, Haemostasis 18, supp.1 pp 7-13 (1988)). References to plasminogen herein are understood to include all these species.

Recently a complete nucleotide sequence has been described (Forsgren, M., et al., 1987, FEBS Letters 213, 254-260). The nucleotide sequence predicts the existence of an extra, previously unreported, isoleucine residue near the N-terminus of the A-chain. This finding has been independently confirmed (McLean, J.N., et al., 1987, Nature 300, 132-137). Accordingly all sequence numbering (amino acid and nucleotide) below follows Forsgren et al. (1987). In this numbering sequence the plasminogen cleavage site is located between residues arg-561 and val-562 and the N-terminal modified forms are termed $met_{69}$, $lys_{78}$ and $val_{79}$.

Plasminogen has five kringle structures. The region from the first to the last cysteine residue of each kringle structure, residues 84 to 162, 166 to 243, 256 to 333, 358 to 435 and 462 to 541 inclusive will be referred to herein as the $K_1^p$, $K_2^p$, $K_3^p$, $K_4^p$ and $K_5^p$ domains respectively.

EP-A-0 213 794 discloses a hybrid plasminogen activator containing plural heterologous polypeptide kringles.

According to the present invention there is provided a hybrid plasminogen activator which comprises one to five kringle domains derived from plasminogen linked to the N-terminus of a t-PA species comprising at least a finger domain and a B-chain derived from t-PA.

The hybrid plasminogen activator (PA) of the invention preferably comprises five kringle domains derived from plasminogen.

The finger domain preferably extends at the N-terminus to include residues preceding residue 6 of native t-PA, such as residues 4 and 5, 1 to 5 or -3 to 5 respectively of the U-, S- or L-chain forms of native t-PA.

The domains may be linked directly by bonds, or by linking sequences of amino acids which may be introduced synthetically during the preparation of the hybrid plasminogen activator (PA) and/or derived from native inter-domain sequences.

Native plasminogen includes a cysteine residue at position 548, C-terminal to plasminogen kringle 5, which participates in an interchain disulphide bridge of the two-chain plasmin form. In the preferred embodiment this residue is deleted or replaced by another residue such as serine.

It will be appreciated that to prevent cleavage of the plasminogen kringle(s) from one another or from t-PA finger domain in vivo, linking sequences may be chosen so as to avoid the presence of a site susceptible to trypsin-like proteolytic cleavage. Thus, in particular, the linking sequences may preferably end with a residue other than arginine or lysine.

The hybrid PA preferably comprises kringles 1 to 5 of plasminogen in sequence.

The hybrid PA may be represented symbolically as:

$$(X-)_m(K^p-Y-)_n F-Z-B$$

where F and B are as defined above, m is 0 or 1, n is an integer of 1 to 5, X comprises the sequence of residues preceding residue 84 of native plasminogen, each of the n values of $K^p$ represents a kringle domain derived from plasminogen, each of the n values of Y independently represents a bond or a linking sequence of amino acids which may be introduced synthetically during the preparation of the hybrid PA and/or derived from native inter-domain plasminogen or t-PA sequences and Z represents a bond or a linking sequence of amino acids which may optionally comprise one or more domains derived from t-PA.

When m is 1, the sequence X may take one of the values found in various forms of native plasminogen, such as the glu$_1$ or lys$_{78}$ forms, that is plasminogen residues 1 to 83 or 78 to 83 respectively.

In a preferred aspect, n is 5 and the hybrid PA may be represented symbolically as:

$$X-K_1^p-Y_1-K_2^p-Y_2-K_3^p-Y_3-K_4^p-Y_4-K_5^p-Y_5-F-Z-B$$

where $Y_1$, $Y_2$, $Y_3$ and $Y_4$ represent bonds or linking sequences, preferably the native plasminogen inter-domain sequences between plasminogen kringle domains 1 and 2, 2 and 3, 3 and 4 and 4 and 5, respectively, Z represents a bond or linking sequence, preferably comprising the kringle 1 and 2 domains and optionally the growth factor domain of native t-PA, $Y_5$ is a bond or a linking sequence and the remaining symbols are defined above.

Where n is 5, preferred sequences ($Y_5$) linking the C-terminal plasminogen kringle domain to the t-PA finger domain are:

[AAPGTRSYQVI] and [AAPSFDSGSYQVI] (single letter amino acid notation). The first sequence consists of residues 542 to 544 of plasminogen, the tripeptide -glycine-threonine-arginine- and residues 1 to 5 of t-PA. The second sequence consists of residues 542 to 547 of plasminogen, the dipeptide -serine-glycine- and residues 1 to 5 of t-PA. The cysteine residue at position 548 of plasminogen has been replaced by serine, and the adjacent glycine residue can be identified with gly-549 of plasminogen.

The sequence Z preferably comprises the G, $K_1^t$ and $K_2^t$ or $K_1^t$ and $K_2^t$ domains of native t-PA.

Preferred compounds of the invention have the following structures:

Plg 1-541[AAPGTRSYQVI]F[HSVPVKS]G[EIDTRAT]$K_1^t$[SEGNSD] $K_2^t$[STCGLRQYSQPQFR]B(Compound 1)
Plg 1-541[AAPSFDSGSYQVI]F[HSVPVKSTRAT]$K_1^t$[SEGNSD] $K_2^t$[STCGLRQYSQPQFR]B(Compound 2) where Plg 1-541 represents residues 1-541 of plasminogen (that is, including kringles 1 to 5), F, G, $K_1^t$, $K_2^t$ and B are as previously defined and the symbols in brackets represent amino acid residues according to the single letter amino acid notation.

The hybrid PA of the invention may be derivatised to provide pharmaceutically useful conjugates analogous to known PA-containing conjugates, for example:

(a) an enzyme-protein conjugate as disclosed in EP-A-O 155 388, in which the catalytic site on the enzyme which is responsible for fibrinolytic activity is blocked by a human protein attached thereto by way of a reversible linking group;

(b) an enzyme-protein conjugate as disclosed in EP-A-O 152 736, comprising at least one optionally blocked fibrinolytic enzyme linked by way of a site other than the catalytic site responsible for fibrinolytic activity to at least one human protein;

(c) a protein-polymer conjugate as disclosed in EP-A-0183503 comprising a pharmaceutically useful protein linked to at least one water soluble polymer by means of a reversible linking group; or

(d) an enzyme conjugate as disclosed in EP-A-0184363 comprising a plurality of fibrinolytic enzymes linked together through the active centres thereof by means of a removable blocking group.

The hybrid PA of the invention may take the place of a PA as the enzyme or (human) protein component, as appropriate, of any of the conjugates described above.

The hybrid PA of the invention or conjugate thereof can be further derivatised such that any catalytic site essential for fibrinolytic activity is optionally blocked by a removable blocking group.

The above mentioned derivatives of the hybrid PA may be used in any of the methods and compositions described hereinafter for the hybrid PA itself.

As used herein the expression 'removable blocking group' includes groups which are removable by hydrolysis at a rate such that the pseudo-first order rate constant for hydrolysis is in the range of $10^{-6}$ sec$^{-1}$ to $10^{-2}$ sec$^{-1}$ in isotonic aqueous media at pH 7.4 at 37°C.

Such blocking groups are described in European Patent No.0009879 and include acyl groups such as optionally substituted benzoyl or optionally substituted acryloyl.

Suitable optional substituents for benzoyl blocking groups include halogen, $C_{1-6}$ alkyl, $C_{1-6}$ alkoxy, $C_{1-6}$ alkanoyloxy, $C_{1-6}$ alkanoylamino, amino or p-guanidino.

Suitable optional substituents for acryloyl blocking groups include $C_{1-6}$ alkyl, furyl, phenyl or $C_{1-6}$ alkylphenyl.

In a further aspect, the invention provides a process for preparing hybrid plasminogen activator according to the invention which process comprises expressing DNA encoding said hybrid plasminogen activator in a recombinant host cell and recovering the hybrid plasminogen activator product.

The DNA polymer comprising a nucleotide sequence that encodes the hybrid PA also forms part of the invention.

The process of the invention may be performed by conventional recombinant techniques such as described in Maniatis et. al., Molecular Cloning - A Laboratory Manual; Cold Spring Harbor, 1982.

In particular, the process may comprise the steps of:

i) preparing a replicable expression vector capable, in a host cell, of expressing a DNA polymer comprising a nucleotide sequence that encodes said hybrid plasminogen activator;

ii) transforming a host cell with said vector;

iii) culturing said transformed host cell under conditions permitting expression of said DNA polymer to produce said hybrid plasminogen activator; and

iv) recovering said hybrid plasminogen activator.

The invention also provides a process for preparing the DNA polymer by the condensation of appropriate mono-, di- or oligomeric nucleotide units.

The preparation may be carried out chemically, enzymatically, or by a combination of the two methods, in vitro or in vivo as appropriate. Thus, the DNA polymer may be prepared by the enzymatic ligation of appropriate DNA fragments, by conventional methods such as those described by D. M. Roberts et al in Biochemistry 1985, 24, 5090-5098.

The DNA fragments may be obtained by digestion of DNA containing the required sequences of nucleotides with appropriate restriction enzymes, by chemical synthesis, by enzymatic polymerisation, or by a combination of these methods.

Digestion with restriction enzymes may be performed in an appropriate buffer at a temperature of 20°-70°C, generally in a volume of 50μl or less with 0.1-10μg DNA.

Enzymatic polymerisation of DNA may be carried out in vitro using a DNA polymerase such as DNA polymerase I (Klenow fragment) in an appropriate buffer containing the nucleoside triphosphates dATP, dCTP, dGTP and dTTP as required at a temperature of 10°-37°C, generally in a volume of 50μl or less.

Enzymatic ligation of DNA fragments may be carried out using a DNA ligase such as T4 DNA ligase in an appropriate buffer at a temperature of 4°C to ambient, generally in a volume of 50μl or less.

The chemical synthesis of the DNA polymer or fragments may be carried out by conventional phosphotriester, phosphite or phosphoramidite chemistry, using solid phase techniques such as those described in 'chemical and Enzymatic Synthesis of Gene Fragments - A Laboratory Manual' (ed. H.G. Gassen and A. Lang), Verlag Chemie, Weinheim (1982),or in other scientific publications, for example M.J. Gait, H.W.D. Matthes, M. Singh, B.S. Sproat, and R.C. Titmas, Nucleic Acids Research, 1982, 10, 6243; B.S. Sproat and W. Bannwarth, Tetrahedron Letters, 1983, 24, 5771; M.D. Matteucci and M.H Caruthers, Tetrahedron Letters, 1980, 21, 719; M.D. Matteucci and M.H. Caruthers, Journal of the American Chemical Society, 1981, 103, 3185; S.P. Adams et al., Journal of the American Chemical Society,1983, 105, 661; N.D. Sinha, J. Biernat, J. McMannus, and H. Koester, Nucleic Acids Research, 1984, 12, 4539; and H.W.D. Matthes et al., EMBO Journal, 1984, 3, 801.

The DNA polymer is preferably prepared by ligating two or more DNA molecules which together comprise a DNA sequence encoding the hybrid PA.

The DNA molecules may be obtained by the digestion with suitable restriction enzymes of vectors carrying the required coding sequences.

In the preferred embodiment where the hybrid PA comprises kringles 1 to 5 of plasminogen, the DNA molecule may conveniently be prepared by ligating a first DNA molecule encoding the required kringles of plasminogen with one or more DNA molecules which, together with said first DNA molecule, encode the t-PA species comprising at least a finger domain and a B-chain derived from t-PA.

Convenient strategies for preparing preferred compounds of the invention are as follows:

## Strategy 1

The first DNA molecule may be obtained by the digestion of a first vector carrying the required part of the plasminogen coding sequence. Conveniently the plasminogen sequence is modified to introduce a new BglII site 3′ to the K₅ᵖ coding region. Digestion of the first vector with restriction enzyme BglII thus provides a DNA molecule coding for plasminogen kringles 1 to 5 and carrying vector functions.

A second DNA molecule may be obtained by the digestion of a second vector carrying the t-PA species coding sequence, with restriction enzyme BglII. Ligation of the first and second DNA molecules will yield a vector carrying DNA coding for a hybrid PA comprising kringles 1 to 5 of plasminogen linked to the N-terminus of a t-PA species.

The nucleotide sequence at the reconstructed BglII site is AGA TCT which codes for residues -arg-ser-. The arginine residue is potentially a site susceptible to in vivo proteolytic cleavage. In order to avoid the presence of such a site, the alternative strategy 2 described hereinafter is preferred.

The vector carrying the required part of the plasminogen coding sequence, that is, kringles 1 to 5 and with a BglII site 3′ thereof may conveniently be obtained by the digestion, separately, with restriction enzymes HindIII and AhaII, and AhaII and DdeI of a vector carrying the plasminogen gene.

The HindIII site is located in the 5′ untranslated region, and the AhaII site at nucleotide position 1572 and the DdeI site at nucleotide position 1748 in the kringle 5 coding region of the plasminogen gene. The HindIII-AhaII and AhaII-DdeI fragments may be ligated together and to an oligonucleotide linker having a DdeI and a BglII sticky end, coding for the remainder of the kringle 5 coding region.

The resulting DNA molecule, coding for plasminogen kringles 1 to 5, has a HindIII and a BglII sticky end and may be held in a suitable vector such as pTRE12 (EP-A-0 201 153) between the HindIII and BglII sites thereof.

## Strategy 2

The first DNA molecule may be obtained by the digestion of a first vector carrying the required part of the plasminogen coding sequence. Conveniently the native plasminogen sequence is modified to introduce a new NarI site 3′ to the K₅ᵖ coding region, preferably at nucleotide position 1758 (Forsgren, M. et al., 1987, op. cit.), and any NarI site formerly present in the vector is destroyed. The first vector preferably also carries at least part of the t-PA coding sequence including the SstI site at nucleotide position 1417 (Pennica et al., 1983, op. cit.) in the B-chain coding region and the portion of the B-chain coding region 3′ thereof. Digestion of the first vector with restriction enzymes NarI and SstI thus provides a DNA molecule coding for plasminogen kringles 1 to 5 and part of the t-PA B-chain, and carrying vector functions.

A second DNA molecule may be obtained by the digestion of a second vector carrying the t-PA species coding sequence, including at least that part of the t-PA B-chain coding sequence 5′ to the SstI site in the B-chain. Digestion of the second vector with restriction enzymes BglII and SstI thus provides a DNA molecule coding for the t-PA species A-chain and part of the B-chain 5′ to the SstI site.

The first and second DNA molecules are linked via an oligonucleotide linker having suitable sticky ends, preferably a 5′ NarI sticky end and a 3′ BamHI sticky end, conveniently of the following structure:

```
5'  CG CCT TCA TTT GAT TCC G          3'
3'     GGA AGT AAA CTA AGG CCT AG      5'
```

The ligation of the first and second DNA molecules with the above linker may be carried out sequentially or, more preferably, in a single step. Ligation of the SstI sticky ends of the molecules reforms the t-PA B-chain coding region. The resulting sequence at the reconstructed NarI site and the BamHI/BglII junction is believed to be as follows:

```
          NarI                    BamHI/BglII junction
5'  ...GCG GCG CCT TCA TTT GAT TCC GGA TCT TAC... 3'
3'  ...CGC CGC GGA AGT AAA CTA AGG CCT AGA ATG... 5'
        ala ala pro ser phe asp ser gly ser tyr
        542 543 544 545 546 547  *  549   1   2
```

Amino acids marked 541-546 are the C-terminal residues of a peptide sequence comprising residues 1 to 547 of plasminogen. The serine residue marked * has been introduced by the use of the linker, and takes the place of cysteine-548 in plasminogen. The glycine residue marked 549 can be identified with residue 549 of plasminogen. Amino acids marked 1 and 2 are the N-terminal residues of the t-PA species.

The said first vector carrying the required part of the plasminogen coding sequence, that is, kringles 1 to 5, and with a NarI site introduced at nucleotide position 1758 may conveniently be obtained by the digestion with restriction enzymes HindIII and AhaII and, separately, AhaII and DdeI of a vector carrying the plasminogen gene, in which any existing vector NarI site has been removed by conventional methods.

The HindIII-AhaII and AhaII-DdeI fragments may be ligated together and to an oligonucleotide linker having a DdeI sticky end, coding for the remainder of the plasminogen kringle 5 coding region and containing a NarI site. The linker is preferably also provided with a BglII sticky end, such that the resulting DNA molecule, coding for plasminogen kringles 1 to 5, has a HindIII and a BglII sticky end and may be held in a suitable vector such as pTRE12 between the HindIII and BglII sites thereof, creating vector A.

In order to introduce the part of the t-PA B-chain coding sequence which is 3' to the SstI site into the vector A which carries the plasminogen $K_1^P$-$K_5^P$ coding region, the BglII fragment encoding the mature t-PA polypeptide obtained from a suitable vector (B) coding therefor, such as pTRE15 (EP-A-0 201 153), is inserted in the BglII site of the vector A. The unwanted portion of the resulting vector may then be excised by digestion with restriction enzymes NarI and SstI to yield the said first DNA molecule encoding plasminogen kringles 1 to 5, t-PA B-chain 3' to the SstI site and vector functions.

The expression of the DNA polymer encoding the hybrid PA in a recombinant host cell may be carried out by means of a replicable expression vector capable, in the host cell, of expressing the DNA polymer. The expression vector is novel and also forms part of the invention.

The replicable expression vector may be prepared in accordance with the invention, by cleaving a vector compatible with the host cell to provide a linear DNA segment having an intact replicon, and combining said linear segment with one or more DNA molecules which, together with said linear segment, encode the hybrid PA, under ligating conditions.

The ligation of the linear segment and more than one DNA molecule may be carried out simultaneously or sequentially as desired.

Thus, the DNA polymer may be preformed or formed during the construction of the vector, as desired.

The choice of vector will be determined in part by the host cell, which may be prokaryotic or eukaryotic, such as mouse C127, mouse myeloma, chinese hamster ovary or yeast. Suitable vectors include plasmids, bacteriophages, cosmids and recombinant viruses.

The preparation of the replicable expression vector may be carried out conventionally with appropriate enzymes for restriction, polymerisation and ligation of the DNA, by procedures described in, for example, Maniatis et al cited above. Polymerisation and ligation may be performed as described above for the preparation of the DNA polymer. Digestion with restriction enzymes may be performed in an appropriate buffer at a temperature of 20°-70°C, generally in a volume of 50µl or less with 0.1-10µg DNA.

The recombinant host cell is prepared, in accordance with the invention, by transforming a host cell with a replicable expression vector of the invention under transforming conditions. Suitable transforming conditions are conventional and are described in, for example, Maniatis et al cited above, or "DNA Cloning" Vol. II, D.M. Glover ed., IRL Press Ltd, 1985.

The choice of transforming conditions is determined by the host cell. Thus, a bacterial host such as E. coli may be treated with a solution of $CaCl_2$ (Cohen et al, Proc. Nat. Acad. Sci., 1973, 69, 2110) or with a solution comprising a mixture of RbCl, $MnCl_2$, potassium acetate and glycerol, and then with 3-[N-morpholino]-propane-sulphonic acid, RbCl and glycerol. Mammalian cells in culture may be transformed by calcium co-precipitation of the vector DNA onto the cells.

The invention also extends to a host cell transformed with a replicable expression vector of the invention.

Culturing the transformed host cell under conditions permitting expression of the DNA polymer is carried out conventionally, as described in, for example, Maniatis et al and "DNA Cloning" cited above. Thus, preferably the cell is supplied with nutrient and cultured at a temperature below 45°C.

The hybrid PA expression product is recovered by conventional methods according to the host cell. Thus, where the host cell is bacterial, such as E. coli it may be lysed physically, chemically or enzymatically and the protein product isolated from the resulting lysate. Where the host cell is mammalian, the product may generally be isolated from the nutrient medium.

The DNA polymer may be assembled into vectors designed for isolation of stable transformed mammalian cell lines expressing the hybrid PA; e.g. bovine papillomavirus vectors (DNA cloning Vol.II D.M. Glover Ed. IRL Press 1985; Kaufman, R.J. et al, Molecular and Cellular Biology 5, 1750-1759, 1985; Pavlakis G.N. and Hamer, D.H., Proceedings of the National Academy of Sciences (USA) 80, 397-401, 1983; Goeddel, D.V. et al.,European Patent Application No. 0093619, 1983).

It will be appreciated that, depending upon the host cell, the hybrid PA prepared in accordance with the invention may be glycosylated to varying degrees. Furthermore, as observed by Pohl et.al., Biochemistry, 1984, 23, 3701-3707, varying degress of glycosylation may also be found in unmodified, naturally occurring t-PA. Plasminogen also exhibits varying degrees of glycosylation (Hayes M.L, J. Biol. Chem. 254: 8768, 1979). Mutant forms of the hybrid PA are also contemplated in which glycosylation sites are removed by genetic engineering techniques. The hybrid PA of the invention is understood to include such glycosylated variations.

It will also be appreciated that, depending upon the expression conditions, the hybrid PA prepared in accordance with the invention may exist in the single or two chain forms or mixtures thereof. The invention extends to all such forms.

The hybrid PA of the invention comprises the B-chain of t-PA linked to a hybrid A-chain comprising one to five kringle domains derived from plasminogen linked to the N-terminus of the A-chain of a t-PA species.

This hybrid PA A-chain may be employed as one chain of a fibrinolytically active hybrid protein such as disclosed in EP-O 155 387. The hybrid A-chain, DNA encoding the hybrid A-chain and a hybrid protein comprising the hybrid A chain linked to the B-chain of a fibrinolytically active protease, the catalytic site of which is optionally blocked by a removable blocking group, all form part of the invention.

The hybrid A-chain may be prepared by separation from the B-chain thereof by mild reduction. Alternatively the hybrid A-chain may be prepared by expressing DNA coding therefor in a recombinant host cell and recovering the hybrid A-chain product. The hybrid protein comprising the hybrid A-chain linked to the B-chain of a fibrinolytically active protease may be prepared by (a) mixing said A- and B-chains under oxidative conditions; or (b) ligating DNA encoding said A-chain to DNA encoding said B-chain and expressing the ligated DNA in a prokaryote or eukaryote host; and thereafter optionally blocking the catalytic site of the hybrid protein with a removable blocking group. The oxidation and reduction conditions are as generally described in EP-A-0 155 387.

The resulting hybrid protein may be used in any of the methods and compositions described hereinafter for the hybrid PA itself.

The hybrid PA of the invention is suitably administered in the form of a pharmaceutical composition.

Accordingly the present invention also provides a pharmaceutical composition comprising hybrid PA of the invention in combination with a pharmaceutically acceptable carrier.

The compositions according to the invention may be formulated in accordance with routine procedures as pharmaceutical compositions adapted for intravenous administration to human beings.

Typically compositions for intravenous administration are solutions of the sterile enzyme in sterile isotonic aqueous buffer. Where necessary the composition may also include a solubilising agent to keep the hybrid PA in solution and a local anaesthetic such as lignocaine to ease pain at the site of injection. Generally, the hybrid PA will be supplied in unit dosage form for example as a dry powder or water free concentrate in a hermetically sealed container such as an ampoule or sachette indicating the quantity of protein in activity units. Where the hybrid PA includes a removable blocking group an indication of the time within which the free protein will be liberated may be given. Where the protein is to be administered by infusion, it will be dispensed with an infusion bottle containing sterile pharmaceutical grade 'Water for Injection' or saline. Where the protein is to be administered by injection, it is dispensed with an ampoule of sterile water for injection or saline. The injectable or infusable composition will be made up by mixing the ingredients prior to administration.

The quantity of material administered will depend upon the amount of fibrinolysis required and the speed with which it is required, the seriousness of the thromboembolic condition and position and size of the clot. The precise dose to be employed and mode of administration must per force in view of the nature of the complaint be decided according to the circumstances by the physician supervising treatment. However, in general, a patient being treated for a thrombus will generally receive a daily dose of from 0.01 to 10 mg/kg of body weight either by injection in for example up to five doses or by infusion.

Within the above indicated dosage range, no adverse toxicological effects are indicated with the compounds of the invention.

Accordingly, in a further aspect of the invention there is provided a method of treating thrombotic diseases, which comprises administering to the sufferer an effective non-toxic amount of hybrid PA of the invention.

The invention also provides hybrid PA of the invention for use as an active therapeutic substance and in particular for use in the treatment of thrombotic diseases.

The following Examples illustrate the invention.

General Methods for Examples

DNA cleavage
In general the cleavage of about 1µg of plasmid DNA or DNA fragments is effected using about 5 units of a restriction enzyme (or enzymes) in about 20µl of an appropriate buffer solution.

Generation of blunt ends:
If blunt ends are required they are produced by treating the DNA preparation with DNA Polymerase I, Klenow fragment (Molecular Cloning, A Laboratory Manual, Cold Spring Harbor Laboratory 1982).

Ligation of DNA Fragments:
Ligation reactions are carried out as described (Maniatis et al Molecular Cloning - A Laboratory Manual, Cold Spring Harbor Laboratory 1982).

Transformation
of plasmid DNA into E. coli HB101 cells is either as described by Hanahan (DNA Cloning Vol I Chapter 6, D.M. Glover ed. IRL Press, 1985) in Protocol 3 except that incubation with RFI is for 5 minutes, or uses competent HB101 cells supplied by Gibco BRL (Paisley, Scotland), used according to the manufacturers instructions.

**Plasmid preparation:**
Large scale preparation of plasmid DNA and plasmid mini-preparations are carried out as described in Maniatis et al., (Molecular Cloning - A Laboratory Manual, Cold Spring Harbor Laboratory, (1982).

**Isolation of DNA fragments from low-melting-point (LMP) agarose gels**
DNA fragments are isolated from LMP agarose gels as described by Maniatis et al., (Molecular Cloning, A Laboratory Manual, 1982, Cold Spring Harbor).

**Oligonucleotides**
Oligonucleotides are made on an Applied Biosystems 381A DNA Synthesizer according to the manufacturers instructions.

**Ligation of Synthetic Linkers to DNA**
Synthetic linkers encoding restriction enzyme sites, are kinased and ligated to blunt-ended DNA as described by Maniatis et al., (Molecular Cloning, A Laboratory Manual, 1982, Cold Spring Harbor).

**Expression of plasminogen activators**
Cell preparation: cells are trypsinised and plated out at $5.4 \times 10^5$ cells per 6cm dish and left in growth medium (10% Serum (021-6010), 1% stock solution of penicillin/streptomycin (043-5070), 2% sodium bicarbonate (043-5080), 1% stock Glutamine (043-5030) in RPMI 1640 medium (041-2400); Gibco, Paisley, Scotland) at 37°C in a humidified incubator in an atmosphere of 5% $CO_2$/95% air. After 72h the cells are re-fed, and used for DNA transfection after a further 24h.
Transfection procedure: The transfection (in Eagles MEM) uses calcium coprecipitation as described in 'DNA Cloning' Ed. D.M. Glover (Chap. 15, C. Gorman). Glycerol shock and 5mM butyrate treatments are used. Plasminogen activator secreted by transfected cells is harvested in RPMI 1640 medium (as above but serum-free, containing 4% soybean peptone (Sigma)) for 24h and activity is measured using fibrin plates with reference to normal t-PA (J.H. Robinson, Thromb. Res., 1984; 33, 155).

**Example 1**

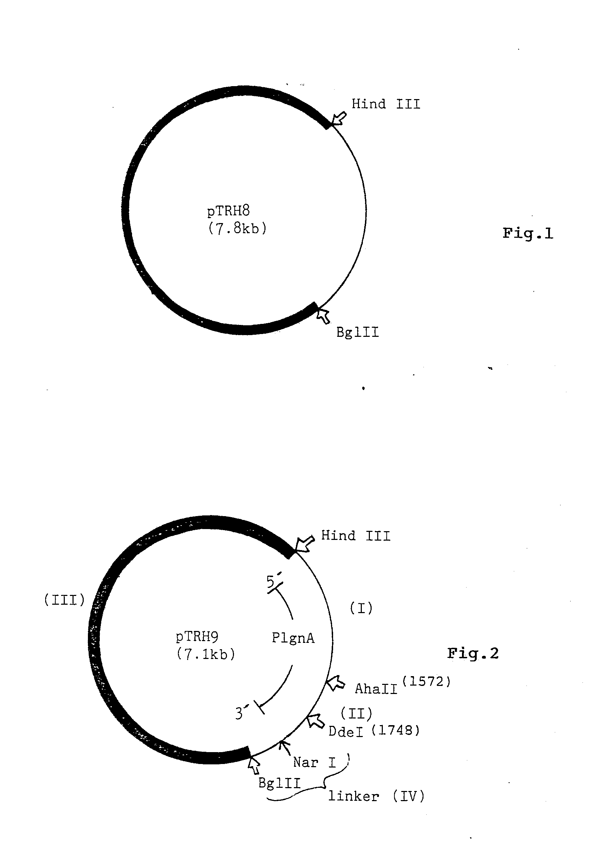

**Construction of an expression plasmid carrying plasminogen cDNA**
A plasminogen cDNA clone is obtained from a human liver cDNA library after screening with an oligonucleotide probe of sequence (A):
5' CC CCT CAC ACA CAT AAC AGG 3'    (A)
corresponding to nucleotides 49 to 68 of the sequence described in Malinowski et al Biochemistry 23, p4243 (1984) (nucleotides 983 to 1002 according to Forsgren, M. et al., 1987).
A plasminogen cDNA clone and associated 5' untranslated regions is subcloned into the vector pTRE12 (EP-A-0 201 153) between the HindIII and BglII sites to create plasmid pTRH5. The single NarI site in plasmid pTRH5 is destroyed by restriction cutting, blunt ending and religation creating plasmid pTRH8 (Fig.1). Removal of the NarI site aids further manipulation.

**Example 2**

**Construction of hybrid plasminogen: t-PA cDNA molecules**
(All plasminogen nucleotide numbering hereafter corresponds to Forsgren et al., 1987, op. cit.)

**(i) Plg 1-541[AAPGTRSYQVI]F[HSVPVKS]G[EIDTRAT]K₁ᵗ[SEGNSD]K₂ᵗ[STCGLRQYSQPQFR]B (Compound 1)**
Three fragments of pTRH8 are prepared by restriction digestion and agarose gel electrophoresis. These fragments are as follows:-
  I: approximately 1.6kb: from a HindIII plus AhaII digest (HindIII [5' to the plasminogen coding sequence] to AhaII[1572]).
  II: approximately 0.18kb: from an AhaII plus DdeI digest (AhaII[1572] to DdeI[1748]).
  III: approximately 5.3kb: largest fragment from a HindIII plus BglII digest, carries the vector functions of pTRH8.
These fragments (I, II, III) are ligated with a kinased oligonucleotide linker (linker IV) comprising two oligonucleotides of sequence:-5' TCAGTGTGCGGCGCCTGGTACCA 3'    (B)
5' GATCTGGTACCAGGCGCCGCACAC 3'    (C)
After transformation into E. coli HB101 the plasmid pTRH9 (Fig.2) may be isolated.
The DNA in plasmid pTRH9 encodes most of the plasminogen A chain, up to and including proline 544 i.e. kringles 1 to 5; the DNA encoding the plasminogen B chain and the remainder of the A chain are absent. Use of linker IV in construction of pTRH9 introduces a unique NarI site, as may be seen from Table 1 which shows the junction of fragments II and III and linker IV.

## TABLE I

Junction of fragments II and III and linker IV in plasmid pTRH9.

```
·· —Fragment II—><——————Linker IV——————————> :Fragment III
            DdeI              NarI              BglII
        1745        1755          1765
5'... GAT GTC CCT CAG TGT GCG GCG* CCT GGT ACC AGA TCT ...3'
3'... CTA CAG GGA GTC ACA CGC CGC  GGA CCA TGG TCT AGA ...5'
      asp val pro gln cys ala ala  pro
      537 538 539 540 541 542 543  544
```

The plasminogen amino-acid sequence is shown

*This residue is C in plasminogen cDNA

Nucleotide numbering is included (above the sequence)

Plasmid pTRH9 is restricted with BglII and ligated with the 2kb BglII fragment encoding the mature t-PA polypeptide isolated from pTRE15 (EP-A-0 201 153).

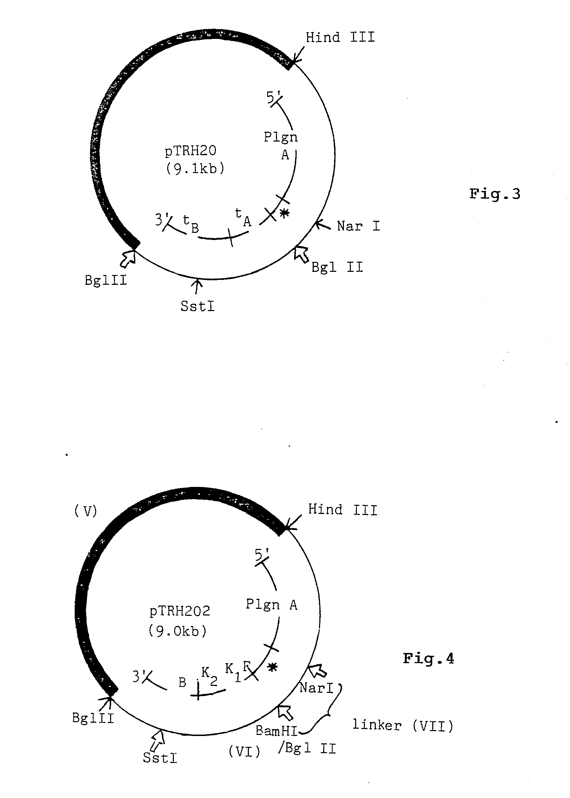

An E. coli HB101 clone carrying the t-PA insert in the same orientation (5' -> 3') as the plasminogen 'A' chain DNA is isolated; this plasmid is called pTRH20 (see Fig 3).

Plasmid pTRH20 encodes a molecule comprising the five kringles of plasminogen (residues 1 to 544) linked to the N-terminus of mature t-PA via the tripeptide gly-thr-arg. The junction of the plasminogen A chain DNA and t-PA A-chain DNA of plasmid pTRH20 is shown in Table 2.

## TABLE 2

Junction of the plasminogen A chain DNA and t-PA A chain DNA in plasmid pTRH20.

```
                                           BglII
5'... CCT CAG TGT GCG GCG CCT GGT ACC AGA TCT TAC CAA ...3'
3'... GGA GTC ACA CGC CGC GGA CCA TGG TCT AGA ATG GTT ...5'
      pro gln cys ala ala pro gly thr arg ser tyr gln
      539 540 541 542 543 544           1   2   3
      <——————————————————  <———————————  ——————————>
          Plasminogen       linking        t-PA
          sequences         tripeptide     sequences
```

Plasmid pTRH20 may be used to transfect human Hela cells (as described in Methods) and produces a novel plasminogen activator.

(ii) Plg 1-541[AAPSFDSGSYQVI]F[HSVPVKSTRAT]K₁ᵗ[SEGNSD] K₂ᵗ[STCGLRQYSQPQFR]B (Compound 2)

The DNA encoding the t-PA A chain and part of the B chain is removed from pTRH20 by restriction with NarI and SstI. The large SstI - NarI fragment (7.9kb) is isolated (fragment V).

An approximately 1.1kb BglII-SstI fragment (fragment VI) is isolated from pTRE24 (EP-A-0207589). This fragment encodes the t-PA fibronectin finger region, kringle 1, kringle 2 and that portion of the t-PA B chain removed from pTRH20 in the process of isolating fragment V.

Two oligonucleotides (D) and (E):

5′ CGCCTTCATTTGATTCCG 3′(D) 5′ GATCCGGAATCAAATGAAGG 3′(E) are made on an Applied Biosystems DNA synthesizer. When kinased and combined these form linker VII.

Fragment VI together with fragment V and linker VII are ligated and transformed into E.Coli HB101. A plasmid pTRH 202 with the structure depicted in Figure 4 may be isolated.

The junction of fragments V and VI and linker VII is shown in Table 3.

## TABLE 3

### Junction of fragments V and VI and linker VII in plasmid pTRH202

```
--- ---Fragment V------->←--linker VII--------------->←Fragment VI---...
      DdeI                NarI                    BamHI/BglII junction

5'...CCT CAG TGT GCG GCG CCT TCA TTT GAT TCC GGA TCT TAC ...3'
3'...GGA GTC ACA CGC CGC GGA AGT AAA CTA AGG CCT AGA ATG ...5'
    pro gln cys ala ala pro ser phe asp ser*gly ser tyr
    539 540 541 542 543 544 545 546 547     549   1   2
                                                   |
                  ←------------------------------------->
                  plasminogen                t-PA
                  sequences                  sequences
```

The plasminogen and t-PA amino-acid sequences at the junction are shown.

*serine in position corresponding to cysteine 548

Plasmid pTRH202 may be used to transfect human Hela cells (as described in Methods) and produces a novel plasminogen activator.

### Example 3

#### Zymography

The product of the transient expression of pTRH20 or pTRH202 may be analysed by fibrin zymography as described by Dodd et al (1986a) Thromb. Haem. 55 (1) 94. The apparent molecular weight observed is consistent with the predicted structure in both cases.

## Example 4

Purification of enzyme encoded by pTRH20

HeLa cells are trypsinised and plated out at a density of $1.4 \times 10^4$ cells per $cm^2$ in 30ml (per $175cm^2$ flask) growth medium (this is: Hepes buffered RPMI 1640 medium (041-2400) containing, 10% Serum (021-6010), 1% stock solution of penicillin/streptomycin (043-5070), 2% sodium bicarbonate solution (043-5080), 1% stock Glutamine (043-5030); Gibco, Paisley, Scotland) at 37°C in a humidified incubator in an atmosphere of 5% $CO_2$/95% air. After 72h an extra 25ml of growth medium is added and the cells used for DNA transfection 24h later.

Transfection procedure: Transfection of plasmid pTRH20 (in Eagles MEM) uses calcium coprecipitation as described in 'DNA Cloning' Ed. D.M. Glover, IRL Press, 1985 (Chap. 15, C. Gorman). Glycerol shock and 5mM butyrate treatment are used. Plasminogen activator secreted by transfected cells is harvested in RPMI 1640 medium (as above) but serum-free, containing 4% soybean peptone (Sigma) for 0-24h and 24-28h (two separate harvests).

pTRH20 protein is purified by chromatography of the 600ml conditioned medium (0-48h harvest) on lysine Sepharose[RTM] 4B. Chromatography conditions are as described previously (Dodd, I. et al (1986b) FEBS Lett. 209 13). The enzyme fraction eluted by the 0.5M arginine-containing buffer is concentrated by stirred-cell ultrafiltration, using YM10 membranes (Amicon), to 1.4ml and buffer-exchanged into 0.05M phosphate/0.1M NaCl/0.01% Tween 80/10mgml$^{-1}$ mannitol/0.05mM ε-aminocaproic acid pH 7.4 (PST/M/E(50μM)) (2.5ml) using a Sephadex G25 (PD10) column.

The purified product solution is assayed on human fibrin plates (Dodd, I. et al, 1986a) against an internal calibration standard standardized against the International Standard for t-PA, Lot 83/517 and against the chromogenic substrate H-D ile-pro-arg-p-nitroanilide (Dodd, I. et al, 1986a). The product is active in both assay systems.

Analysis by sodium dodecyl sulphate polyacrylamide gel electrophoresis followed by fibrin zymography using methods described previously (Dodd, I. et al, 1986a) shows that the product contains a major fibrinolytically active species at apparent $M_r$ 130000. A minor species is present at apparent $M_r$ 65000.

## Example 5

Synthesis and characterisation of N,N-dimethyl-4-aminobenzoyl (DAB) mutant plasminogen activator

Mutant activator purified as described in Example 4 is reversibly blocked at the active centre of the molecule by 4'-amidinophenyl-N,N-dimethyl-4-aminobenzoate.HCl (AP-DAB) using a method similar to that described in Browne, M.J. et al (1988) J. Biol. Chem. 263 1599. Specifically, mutant activator (2.0ml, 6400 IU) is mixed with AP-DAB (4μl, 10mM) and incubated at 25°C for 35 min. The S2288 activity (Dodd I. et al 1986a) of the preparation decreases to 7% of the starting activity. The solution is then buffer-exchanged into PST/M/E(1mM) (3.0ml) and stored at -70°C.

An aliquot of the acylated product is diluted with an equal volume of PST and incubated at 37°C. Deacylation is monitored by measuring the return of S2288 activity. The results indicate that greater than 90 per cent of the product is in the active centre-blocked form, and that the deacylation rate constant is $1.2 \times 10^{-4}$ sec$^{-1}$.

Sephadex and Sepharose are trademarks.

In the Figures:

Figure 1 Structure of pTRH8

```
▭▭▭▭    = pTRE12 sequences

_____    = plasminogen cDNA sequences (Hind III at 5'
              end, BglII at 3' end).

  ◁▷      = restriction sites used in construction
```

Figure 2 Structure of pTRH9 Fragments (I) to (III) and linker (IV) are indicated

```
▨▨▨▨          = pTRE12 Sequences
_____    = insert Sequences
PlgnA         = plasminogen A chain coding region
↩            = restriction sites used in construction
↩            = other restriction sites
▭ ⸺ ⸺,       = protein coding regions
```

Figure 3 Structure of pTRH20

```
▨▨▨▨          = vector sequences
_____    = insert DNA
↩            = restriction sites used in construction
←            = other restriction sites
*             = linking amino acid sequence
▭ ⸺ ⸺ ▭       = protein coding regions
Plgn A        = plasminogen A chain-coding region
```

$t_A$ = t-PA A chain coding region

$t_B$ = t-PA B chain coding region

Figure 4 Structure of pTRH202 Fragments [(V)(SstI-NarI), (VI)(Bgl II-SstI) and linker (VII) (Nar I-BamHI)] are indicated

```
▨▨▨▨          = vector sequences
_____    = insert DNA
↩            = restriction sites used in construction
←            = other restriction sites
*             = linking amino acid sequence
▭⸺⸺⸺▭         = protein coding regions
Plgn A        = plasminogen A chain coding region
F             = fibronectin finger coding region
```

$K_1$ = t-PA kringle 1 coding region

$K_2$ = t-PA kringle 2 coding region

B = t-PA B chain coding region

N.B. The figures are not to scale.

## Claims

1. A hybrid plasminogen activator which comprises one to five kringle domains derived from plasminogen linked to the N-terminus of a t-PA species comprising at least a finger domain and a B-chain derived from t-PA.

2. A hybrid plasminogen activator according to claim 1, which comprises five kringle domains derived from plasminogen.

3. A hybrid plasminogen activator according to claim 2 of the formula:

$$X-K_1^p-Y_1-K_2^p-Y_2-K_3^p-Y_3-K_4^p-Y_4-K_5^p-Y_5-F-Z-B$$

where X comprises the sequence of residues preceding residue 84 of native plasminogen, $Y_1, Y_2, Y_3, Y_4, Y_5$ and Z represent bonds or linking sequences, $K_1^p, K_2^p, K_3^p, K_4^p$ and $K_5^p$ respectively represent each of the five kringle structures of plasminogen, F is the finger domain of t-PA and B is the B-chain of t-PA.

4. A hybrid plasminogen activator according to claim 3, wherein $Y_5$ is

[AAPSFDSGSYQVI]

5. Plg 1-541[AAPGTRSYQVI]F[HSVPVKS]G[EIDTRAT]$K_1^t$ [SEGNSD]$K_2^t$[STCGLRQYSQPQFR]B   or
Plg 1-541[AAPSFDSGSYQVI]F[HSVPVKSTRAT]$K_1^t$[SEGNSD] $K_2^t$[STCGLRQYSQPQFR]B,

where Plg 1-541 represents residues 1-541 of plasminogen, F and B are as defined in claim 3, G represents the growth factor domain of t-PA and $K_1^t$ and $K_2^t$ represent the kringle 1 and kringle 2 domains respectively of t-PA, and the symbols in brackets represent amino acid residues according to the single letter amino acid notation.

6. A hybrid plasminogen activator according to any preceding claim wherein the catalytic site essential for fibrinolytic activity is blocked by a removable blocking group.

7. A pharmaceutical composition comprising a hybrid plasminogen activator according to any preceding claim in combination with a pharmaceutically acceptable carrier.

8. A hybrid plasminogen activator according to any of claims 1 to 6 for use in the treatment of thrombotic diseases.

9. A process for preparing a hybrid enzyme according to claim 1, which process comprises expressing DNA encoding said hybrid plasminogen activator in a recombinant host cell and recovering the hybrid plasminogen activator product.

10. A DNA polymer comprising a nucleotide sequence that encodes a hybrid plasminogen activator according to claim 1.

0292326

pTRH8
(7.8kb)

Hind III

BglII

Fig.1

(III)

pTRH9
(7.1kb)

PlgnA

5'

3'

Hind III

(I)

AhaII (1572)

(II)
DdeI (1748)

Nar I

BglII

linker (IV)

Fig.2

0292326

Fig.3

Fig.4